# EUROPÄISCHE PATENTSCHRIFT

(11) **EP 0 864 565 B1**
(45) Veröffentlichungstag und Bekanntmachung des Hinweises auf die Patenterteilung: **05.06.2002**
(21) Anmeldenummer: 98104312.8
(22) Anmeldetag: 10.03.1998
(51) Int. Cl.: C07D 213/79

(54) **Verfahren zur Herstellung von 2,6-Pyridindicarbonsäureestern**
Process for the preparation of 2,6-pyridinedicarboxylic esters
Procédé pour la préparation d'esters de l'acide 2,6-pyridinedicarboxylique

(30) Priorität: 12.03.1997 CH 59597
(43) Veröffentlichungstag der Anmeldung: 16.09.1998
(73) Patentinhaber: Lonza AG, 3930 Visp (CH)
(72) Erfinder: Roduit, Jean-Paul, Dr., 3979 Grône, Kanton Wallis (CH); Bessard, Yves, Dr., 3960 Sierre (CH)
(74) Vertreter: Winter, Brandl, Fürniss, Hübner, Röss, Kaiser, Polte Partnerschaft

(56) Entgegenhaltungen:
- EP-A- 0 820 986
- WO-A-93/18005
- DE-B- 1 620 174
- GB-A- 2 009 163
- I. IOVEL ET AL.: SYNTHETIC COMMUNICATIONS, Bd. 22, Nr. 18, 1992, Seiten 2691-6, XP002068078
- G. WANG ET AL.: SYNLETT, Nr. 5, 1992, Seiten 422-4, XP002068079

## Beschreibung

Die vorliegende Erfindung betrifft ein Verfahren zur Herstellung von 2,6-Pyridindicarbonsäureestern durch Umsetzung von halogenierten Pyridinen mit Kohlenmonoxid und einem Alkohol in Gegenwart einer Base und eines Katalysators.
2,6-Pyridindicarbonsäureester sind wichtige Zwischenprodukte, z B. für die Herstellung von Verbindungen mit entzündungshemmender Wirkung (JP-A 93 / 143 799).
Die erfindungsgemäss herstellbaren 2,6-Pyridindicarbonsäureester besitzen die allgemeine Formel 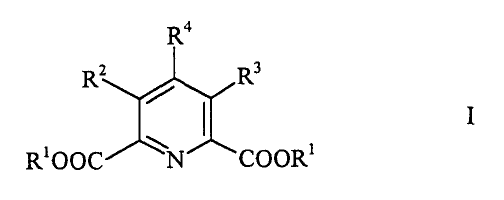 worin R¹ eine C₁-C₆-Alkylgruppe, eine C₃-C₆-Cycloalkylgruppe, eine Arylgruppe oder eine Arylalkylgruppe und R² und R³ unabhängig voneinander Wasserstoff oder Chlor und R⁴ Wasserstoff, eine C₁-C₆ Alkylgruppe, eine C₁-C₆-Alkoxygruppe oder Fluor ist.
Die ältere EP-A-0 820 986 beschreibt ein Verfahren zur Herstellung von 2-Pyridincarbonsäureestern, worin ein 2-Halopyridin mit Kohlenmonoxid und einem Alkohol in Gegenwart einer Base und eines katalytisch wirksamen Pd-Komplexes umgesetzt wird. Bekannte Verfahren zur Herstellung von 2,6-Pyridindicarbonsäureestern basieren auf der direkten chemischen Oxidation von 2,6-Dimethylpyridin (I. Iovel, M. Shymanska, Synth. Commun. 1992, 22, 2691; G. Wang, D.E. Bergstrom, Synlett 1992, 422; BE-A 872 394, SU-A 568 642). Obwohl hierbei teilweise hohe Ausbeuten erzielt werden, sind die Verfahren wegen der Verwendung von teuren und/oder toxischen Reagentien mit Nachteilen verbunden. Verfahren zur Herstellung von 2,6-Pyridindicarbonsäureestern durch Carbonylierung von 2,6-Dichlorpyridin unter Einsatz eines Nickelkatalysators sind bekannt (WO 93/18005). Nachteilig bei diesem Verfahren ist, dass die Reaktion bei hohem Druck durchgeführt wird, die Pyridincarbonsäureester nur in mässigen Ausbeuten erhalten werden und ein hoher Anteil an monocarbonyliertem Nebenprodukt gebildet wird.

Die Aufgabe der vorliegenden Erfindung war daher, ein ökonomisches Verfahren zur Verfügung zu stellen, mit welchem, ausgehend von Halopyridinen, 2,6-Pyridindicarbonsäureester der allgemeinen Formel I in hohen Ausbeuten herzustellen sind Erfindungsgemäss wird diese Aufgabe durch das Verfahren nach Patentanspruch 1 gelöst Darin werden Halopyridine der allgemeinen Formel 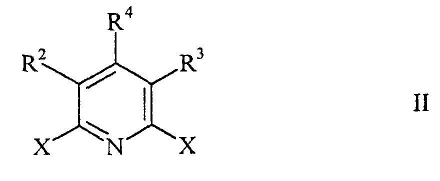 worin R², R³ und R⁴ die oben genannte Bedeutung haben und X Chlor oder Brom ist, mit Kohlenmonoxid und einem Alkohol der allgemeinen Formel

R¹―OH III

worin R¹ die oben genannte Bedeutung hat, in Gegenwart einer Base und eines Komplexes von Palladium mit einem Bis-diphenylphosphin der allgemeinen Formel 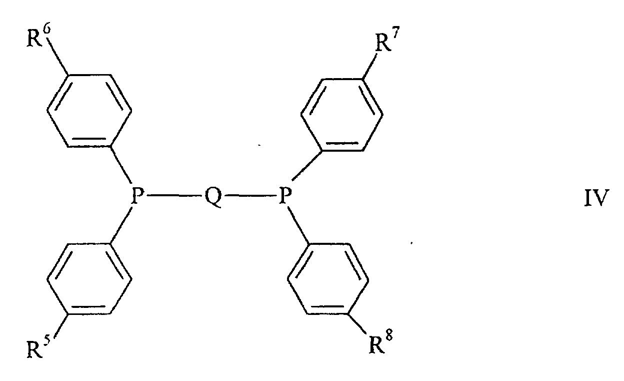 worin Q eine C₃-C₆-Alkandiylgruppe oder eine 1,1'-Ferrocendiylgruppe mit gegebenenfalls mit C₁-C₄-Alkyl- oder Arylgruppen substituierten Cyclopentadienylgruppen und R⁵ bis R⁸ unabhängig voneinander Wasserstoff, C₁-C₄-Alkyl, C₁-C₄-Alkoxy, Monofluormethyl, Difluormethyl, Trifluormethyl, Fluor, Aryl, Aryloxy, Cyano oder Dialkylamino bedeuten, zur Reaktion gebracht wird.
R¹ ist eine geradkettige oder verzweigte Alkylgruppe mit 1 bis 6 C-Atomen, eine Cycloalkylgruppe mit 3 bis 6 C-Atomen, eine Arylgruppe oder eine Arylalkylgruppe. Namentlich erwähnt seien Methyl, Ethyl, Propyl, Isopropyl, Butyl, Isobutyl, tert-Butyl, Pentyl und seine Isomeren sowie Hexyl und seine Isomeren, Cyclopropyl, Cyclobutyl, Cyclopentyl sowie Cyclohexyl. Unter Arylgruppen sind hier insbesondere mono- oder polycyclische Systeme wie beispielsweise Phenyl, Naphthyl oder Anthracenyl oder Biphenylylgruppen zu verstehen. Diese können einen oder mehrere gleiche oder verschiedene Substituenten tragen, beispielsweise niedrige Alkylgruppen, insbesondere mit C₁-C₆ wie Methyl, halogenierte Alkylgruppen wie Trifluormethyl, niedrige Alkoxygruppen, insbesondere mit C₁-C₆ wie Methoxy, oder niedrige Alkylthio- (Alkansulfanyl-) oder Alkansulfonylgruppen, insbesondere C₁-C₆ wie Methylthio oder Ethansulfonyl. Unter substituiertem Phenyl sind hier und im folgenden insbesondere Gruppen wie Fluorphenyl, Methoxyphenyl, Tolyl oder Trifluormethylphenyl zu verstehen, wobei sich die Substituenten vorzugsweise in der para-Position befinden.
Entsprechend sind unter Arylalkyl die aus niedrigen Alkylgruppen, insbesondere C₁-C₆-Alkyl, durch Austausch eines Wasserstoffatoms gegen eine der vorstehend definierten Arylgruppen gebildeten Gruppen zu verstehen, also beispielsweise Benzyl oder Phenylethyl.
Besonders bevorzugt hat R¹ die Bedeutung von Methyl, Ethyl, Butyl und Cyclohexyl.
R² und R³ haben unabhängig voneinander die Bedeutung von Wasserstoff oder Chlor. Besonders bevorzugt bedeuten R² und R³ Wasserstoff. R⁴ ist Wasserstoff, eine geradkettige oder verzweigte Alkylgruppe mit 1 bis 6 C-Atomen oder eine geradkettige oder verzweigte Alkoxygruppe mit 1 bis 6 C-Atomen oder Fluor. Namentlich erwähnt seien Methyl, Ethyl, Propyl, Isopropyl, Butyl, Isobutyl, tert-Butyl, Pentyl und seine Isomeren sowie Hexyl und seine Isomeren, Methoxy, Ethoxy, Propoxy, Isopropoxy, Butoxy, Isobutoxy, tert-Butoxy, Pentyloxy und seine Isomeren sowie Hexyloxy und seine Isomeren. Besonders bevorzugt hat R⁴ die Bedeutung von Wasserstoff. X hat die Bedeutung von Chlor oder Brom, besonders bevorzugt hat X die Bedeutung von Chlor.

Halopyridine der allgemeinen Formel II sind kommerziell erhältlich.

Vorzugsweise werden nach dem erfindungsgemässen Verfahren Methyl-, Ethyl-, Butyl- oder Cyclohexylester hergestellt (R¹ = Methyl, Ethyl, Butyl, Cyclohexyl), indem als Alkohol (III) Methanol, Ethanol, Butanol oder Cyclohexanol eingesetzt wird

Die Umsetzung erfolgt in Gegenwart einer Base. Gut geeignet sind beispielsweise Alkali- und Erdalkaliacetate, -carbonate, -hydrogencarbonate, -phosphate oder -hydrogenphosphate. Namentlich erwähnt seien Natriumacetat, Kaliumacetat, Magnesiumacetat, Calciumacetat, Natriumcarbonat, Kaliumcarbonat, Magnesiumcarbonat, Calciumcarbonat, Natriumhydrogencarbonat, Kaliumhydrogencarbonat, Magnesiumhydrogencarbonat, Calciumhydrogencarbonat, Magnesiumphosphat, Calciumphosphat, Natriumdihydrogenphosphat, Kaliumdihydrogenphosphat, Dinatriumhydrogenphosphat, Dikaliumhydrogenphosphat, Magnesiumhydrogenphosphat, Calciumhydrogenphosphat. Besonders geeignet ist Natriumacetat.

Der katalytisch wirksame Palladium-bis-diphenylphosphin-Komplex wird vorteilhaft *in situ* gebildet, indem ein Pd(II) Salz (z. B. das Chlorid oder das Acetat) oder ein geeigneter Pd(II)-Komplex (z. B. Bis(triphenylphosphin)palladium(II)chlorid) mit dem Diphosphin zur Reaktion gebracht wird. Als Pd(II) Salz oder Pd(II)-Komplex werden bevorzugt Palladium(II)acetat oder Bis(triphenylphosphin)palladium (II)chlorid eingesetzt. Das Palladium wird vorzugsweise in einer Menge von 0,05 bis 0,4 Mol-% Pd(II), bezogen auf die Halogenverbindung (II), eingesetzt. Das Diphosphin wird vorteilhaft im Überschuss (bezogen auf Pd) eingesetzt, vorzugsweise in einer Menge von 0,2 bis 5 Mol-%, ebenfalls bezogen auf die Halogenverbindung (II).

Als Bis-diphenylphosphine (IV) werden vorteilhaft solche eingesetzt, in denen Q eine geradkettige oder verzweigte Alkandiylgruppe mit 3 bis 6 C-Atomen bedeutet. Namentlich erwähnt seien Propan-1,3-diyl, Propan-1,2-diyl, Butan-1,4-diyl, Butan-1,3-diyl, Butan-1,2-diyl, Pentandiyl und seine Isomeren sowie Hexandiyl und seine Isomeren. Vorzugsweise werden solche eingesetzt, in denen Q eine geradkettige Alkandiylgruppe mit 3-6 C-Atomen bedeutet. Namentlich erwähnt seien Propan-1,3-diyl, Butan-1,4-diyl, Pentan-1,5-diyl sowie Hexan-1,6-diyl. Besonders bevorzugt ist 1,4-Bis(diphenyl-phosphino)butan.
Ebenfalls vorteilhaft eingesetzt werden Bis-diphenylphosphine (IV), in denen Q eine 1,1'-Ferrocendiylgruppe mit gegebenenfalls mit C₁-C₄-Alkyl- oder Arylgruppen substituierten Cyclopentadienylgruppen bedeutet. Als C₁-C₄-Alkyl-Substituenten werden bevorzugt Methyl, Ethyl, Propyl, Isopropyl, Butyl, Isobutyl, tert-Butyl eingesetzt, besonders bevorzugt sind Methyl und Ethyl. Als Aryl-Substituenten werden hier bevorzugt Phenyl oder gegebenenfalls substituiertes Phenyl eingesetzt. Besonders bevorzugt wird 1,1'-Bis(diphenylphosphino)-ferrocen eingesetzt.

R⁵ bis R⁸ sind unabhängig voneinander Wasserstoff, C₁-C₄-Alkyl, C₁-C₄-Alkoxy, Monofluormethyl, Difluormethyl, Trifluormethyl, Fluor, Aryl, Aryloxy, Cyano oder Dialkylamino.

Als C₁-C₄-Alkyl-Substituenten werden vorteilhaft Methyl, Ethyl, Propyl, Isopropyl, Butyl, Isobutyl oder tert-Butyl eingesetzt, besonders bevorzugt sind Methyl und Ethyl Als C₁-C₄-Alkoxy-Substituenten werden vorteilhaft Methoxy, Ethoxy, Propoxy, Isopropoxy, Butoxy, Isobutoxy oder tert-Butoxy eingesetzt, besonders bevorzugt sind Methoxy und Ethoxy. Als Aryl-Substituenten werden hier vorteilhaft Phenyl oder gegebenenfalls substituiertes Phenyl eingesetzt. Als Aryloxy-Substituenten werden vorteilhaft Phenoxy und gegebenenfalls substituiertes Phenoxy eingesetzt. Unter substituiertem Phenoxy sind insbesondere Gruppen wie Fluorphenoxy, Methoxyphenoxy, Tolyloxy oder Trifluormethylphenoxy zu verstehen, wobei sich die Substituenten vorzugsweise in der para-Position befinden.
Als Dialkylamino-Substituenten werden bevorzugt Amine mit C₁-C₂-Alkyl-Resten eingesetzt. Namentlich erwähnt seien Dimethylamino, Diethylamino.

Der Alkohol (III) kann auch als Lösungsmittel dienen. Gegebenenfalls kann ein zusätzliches Lösungsmittel eingesetzt werden. Als zusätzliches Lösungsmittel kommen sowohl unpolare, wie beispielsweise Toluol oder Xylol, als auch polare organische Lösungsmittel wie beispielsweise Acetonitril, Tetrahydrofuran oder *N,N*-Dimethylacetamid in Frage.

Die Reaktion wird vorteilhaft bei einer Reaktionstemperatur von 100 bis 250°C, vorzugsweise bei 140 bis 195°C und einem Kohlenmonoxiddruck von vorteilhaft 1 bis 200 bar, vorzugsweise 5 - 50 bar durchgeführt. Nach einer Umsetzungszeit von üblicherweise 1 bis 20 Stunden erhält man die Verbindung der allgemeinen Formel I in hoher Ausbeute

Die folgenden Beispiele verdeutlichen die Durchführung des erfindungsgemässen Verfahrens.

### Beispiel 1

### 2,6-Pyridindicarbonsäuredimethylester

In einem Autoklaven wurden 1,48 g (10 mmol) 2,6-Dichlorpyridin, 166 mg 1,1'-Bis(diphenylphosphino)ferrocen (3 Mol% bezogen auf 2,6-Dichlorpyridin), 4,6 mg Palladium(II)acetat (0,2 Mol% bezogen auf 2,6-Dichlorpyridin), 1,72 g (21 mmol) Natriumacetat und 25 ml Methanol vorgelegt. Der Autoklav wurde mehrmals mit Kohlenmonoxid durchströmt um die Luft gegen Kohlenmonoxid auszutauschen. Anschliessend wurde Kohlenmonoxid mit 15 bar in den Autoklaven gepresst. Das Reaktionsgemisch wurde auf 135 °C (Badtemperatur) erhitzt und 1 Stunde gerührt. Nach dem Abkühlen auf Raumtemperatur wurde das Rohprodukt unter Vakuum (30 mbar) aufkonzentriert und an Kieselgel 60 (Laufmittel Hexan/Ethylacetat 1:1) chromatographiert.
Schmp.: 120,8 - 122,5 °C
Ausbeute: 1,52 g (78%) weisses Pulver
MS; *m z:* 195 (M⁺), 165, 137, 105
¹H-NMR (CDCl₃): δ = 8,31 (d, 2H);
   8,02 (t, 1H);
   4,02 (s, 6H)

### Beispiel 2

### 2,6-Pyridindicarbonsäurediethylester

Es wurde verfahren wie in Beispiel 1 beschrieben, jedoch wurde anstelle von Methanol das gleiche Volumen Ethanol und anstelle von 21 mmol 57 mmol Natriumacetat eingesetzt. Nach einer Reaktionszeit von 1 Stunde bei einer Badtemperatur von 135 °C wurden 1,96 g (88%) weisses Pulver erhalten.
Schmp.. 41,5 - 42,8 °C
MS; *m z*: 224, 223 (M⁺); 208; 179; 151; 123; 105
¹H-NMR (CDCl₃): δ = 8,28 (d, 2H);
   8,00 (t, 1H);
   4,50 (q, 4H);
   1,45 (t, 6H).

### Beispiel 3

### 2,6-Pyridindicarbonsäurediethylester

Es wurde verfahren wie in Beispiel 1 beschrieben, jedoch wurde anstelle von 25 ml Methanol 25 ml Tetrahydrofuran und 30 ml Ethanol eingesetzt. Nach einer Reaktionszeit von 3 Stunden bei einer Badtemperatur von 150 °C wurden 0,56 g (25,1%) weisses Pulver erhalten.

### Beispiel 4

### 2,6-Pyridindicarbonsäurediethylester

Es wurde verfahren wie in Beispiel 1 beschrieben, jedoch wurde anstelle von Methanol das gleiche Volumen Ethanol und anstelle von 4,6 mg Palladium(II)acetat 14,0 mg Bis(triphenylphosphin)palladium(II)chlorid eingesetzt. Nach einer Reaktionszeit von 2 Stunden bei einer Badtemperatur von 175 °C wurden 1,54 g (69%) weisses Pulver erhalten.

### Beispiel 5

### 2,6-Pyridindicarbonsäurediethylester

Es wurde verfahren wie in Beispiel 2 beschrieben, jedoch wurde anstelle von 166 mg 1,1'-Bis(diphenylphosphino)ferrocen 128 mg 1,4-Bis(diphenylphosphino)butan eingesetzt. Nach einer Reaktionszeit von 3 Stunden bei einer Badtemperatur von 150 °C wurden 1,64 g (73,5%) weisses Pulver erhalten.

### Beispiel 6

### 2,6-Pyridindicarbonsäuredibutylester

Es wurde verfahren wie in Beispiel 1 beschrieben, jedoch wurde anstelle von Methanol das gleiche Volumen Butanol eingesetzt. Nach einer Reaktionszeit von 1 Stunde bei einer Badtemperatur von 135 °C wurden 2,45 g (85%) weisses Pulver erhalten.
Schmp.: 65,5 - 65,9 °C
MS; *m z*: 280 (M⁺); 236; 224; 206; 179; 150; 123; 105; 78
¹H-NMR (CDCl₃): δ = 8,28 (d, 2H);
   7,98 (t, 1H);
   5,32 (sept, 2H);
   4,42 (t, 4H);
   1,82 (quint, 4H);
   1,50 (sext, 4H);
   0,99 (t, 6H).

### Beispiel 7

### 2,6-Pyridindicarbonsäuredicyclohexylester

Es wurde verfahren wie in Beispiel 1 beschrieben, jedoch wurde anstelle von Methanol das gleiche Volumen Cyclohexanol eingesetzt. Nach einer Reaktionszeit von 1 Stunde bei einer Badtemperatur von 135 °C wurden 1,7 g (51%) weisses Pulver erhalten.
Schmp.: 111,6 - 112,3 °C
MS; *m z*: 331 (M⁺); 287; 250; 219; 205; 168; 150; 123
¹H-NMR (CDCl₃): δ = 8,22 (d, 2H);
   7,97 (t, 1H);
   5,10 (sept, 2H);
   2,1 - 1,3 (m, 22H).

### Beispiel 8

### 3-Chlor-2,6-pyridindicarbonsäurediethylester

Es wurde verfahren wie in Beispiel 2 beschrieben, jedoch wurde anstelle von 1,48 g (10 mmol) 2,6-Dichlorpyridin 1,82 g (10 mmol) 2,3,6-Trichlorpyridin eingesetzt. Nach einer Reaktionszeit von 1 Stunde bei einer Badtemperatur von 135 °C wurden 1,98 g (76%) farbloses Öl erhalten.
MS (*m*/*z*): 257 (M⁺); 213; 185; 139; 113.
1H NMR (CDCl₃) δ = 8,15 (d, 1H);
   7,93 (d, 1H);
   4,50 (q, 2H);
   4,48 (d) 2H);
   1,44 (t, 3H);
   1,43 (t, 3H).

## Patentansprüche

1. Verfahren zur Herstellung von 2,6-Pyridindicarbonsäureestern der allgemeinen Formel 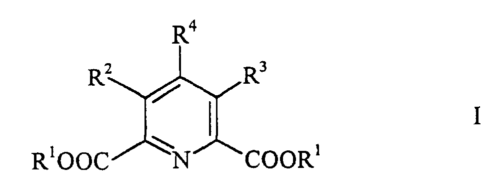 worin R¹ eine C₁-C₆-Alkylgruppe, eine C₃-C₆-Cycloalkylgruppe oder eine gegebenenfalls mit C₁-C₆-Alkyl, halogeniert oder unhalogeniert, C₁-C₆-Alkoxy, C₁-C₆-Alkylthio, C₁-C₆-Alkansulfonyl oder Fluor substituierte Aryl- oder Arylalkylgruppe und R² und R³ unabhängig voneinander Wasserstoff oder Chlor bedeuten und R⁴ Wasserstoff, eine C₁-C₆-Alkylgruppe, eine C₁-C₆-Alkoxygruppe oder Fluor ist, **dadurch gekennzeichnet, dass** ein Halopyridin der allgemeinen Formel 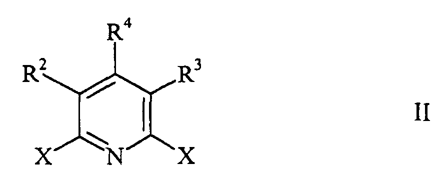 worin R², R³ und R⁴ die oben genannte Bedeutung haben und X Chlor oder Brom ist, mit Kohlenmonoxid und einem Alkohol der allgemeinen Formel
R¹―OH III
worin R¹ die oben genannte Bedeutung hat, in Gegenwart einer Base und eines Komplexes von Palladium mit einem Bis-diphenylphosphin der allgemeinen Formel 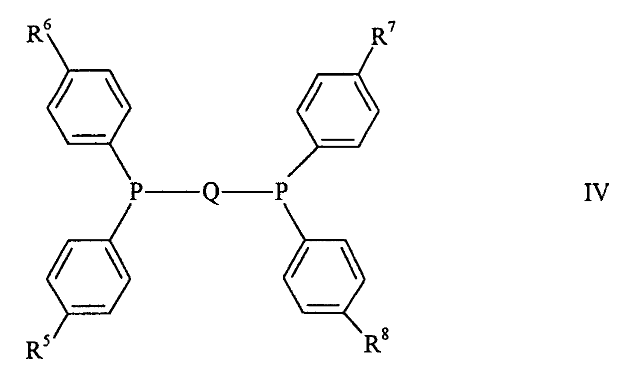 worin Q eine C₃-C₆-Alkandiylgruppe oder eine 1,1'-Ferrocendiylgruppe mit gegebenenfalls mit C₁-C₄-Alkyl- oder Arylgruppen substituierten Cyclopentadienylgruppen und R⁵ bis R⁸ unabhängig voneinander Wasserstoff, C₁-C₄-Alkyl, C₁-C₄-Alkoxy, Monofluormethyl, Difluormethyl, Trifluormethyl, Fluor, Aryl, Aryloxy, Cyano oder Dialkylamino bedeuten, zur Reaktion gebracht wird.

2. Verfahren nach Anspruch 1, **dadurch gekennzeichnet, dass** R¹ eine Methylgruppe, Ethylgruppe, Butylgruppe oder Cyclohexylgruppe ist.

3. Verfahren nach Anspruch 1 oder 2, **dadurch gekennzeichnet, dass** die Base ein Alkalioder Erdalkaliacetat, -carbonat, -hydrogencarbonat, -phosphat oder -hydrogenphosphat ist.

4. Verfahren nach einem der Ansprüche 1 bis 3, **dadurch gekennzeichnet, dass** Palladium in Form von Bis(triphenylphosphin)palladium(II)chlorid oder Palladium(II)acetat eingesetzt wird.

5. Verfahren nach einem der Ansprüche 1 bis 4, **dadurch gekennzeichnet, dass** das Bis-diphenylphosphin 1,1'-Bis(diphenylphosphino)ferrocen oder 1,4-Bis(diphenylphosphino)butan ist.

6. Verfahren nach einem der Ansprüche 1 bis 5, **dadurch gekennzeichnet, dass** der Kohlenmonoxiddruck 1 bis 200 bar ist.

7. Verfahren nach einem der Ansprüche 1 bis 6, **dadurch gekennzeichnet, dass** die Reaktionstemperatur 100 bis 250 °C ist.

## Claims

1. Process for the production of 2,6-Pyridinedicarboxylic acid esters of the general formula 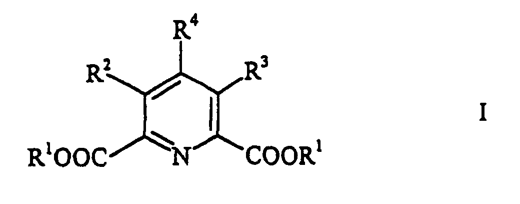 wherein R¹ means a C₁-C₆-alkyl group, a C₃-C₆-cycloalkyl group or an aryl- or aralkyl group, optionally substituted with C₁-C₆-alkyl, halogenated or unhalogenated, C₁-C₆-alkoxy, C₁-C₆-alkylthio, C₁-C₆-alkanesulfonyl or fluoro and R² and R³ independently of each other mean hydrogen or chlorine and R⁴ is hydrogen, a C₁-C₆-alkyl group, a C₁-C₆-alkoxy group or fluorine, **characterised in that** a halopyridine of the general formula 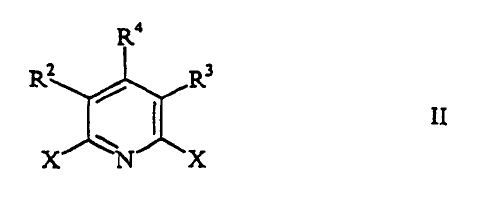 wherein R², R³ and R⁴ have the above meaning and X is chlorine or bromine, is reacted with carbon monoxide and an alcohol of the general formula
R¹―OH III
wherein R¹ has the above meaning, in the presence of a base and a complex of palladium with a bisdiphenylphosphine of the general formula 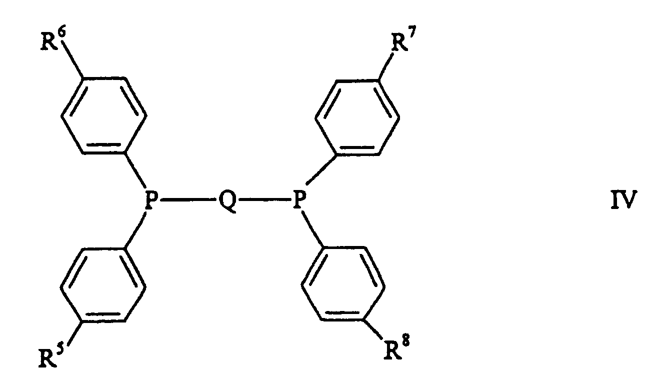 wherein Q means a C₃-C₆-alkanediyl group or a 1,1'-ferrocenediyl group with cyclopentadienyl groups optionally substituted with C₁-C₄-alkyl or -aryl groups and R⁵ to R⁸, independently of each other, mean hydrogen, C₁-C₄-alkyl, C₁-C₄-alkoxy, monofluoromethyl, difluoromethyl, trifluoromethyl, fluoro, aryl, aryloxy, cyano or dialkylamino.

2. Process according to claim 1, **characterised in that** R¹ is a methyl group, ethyl group, butyl group or cyclohexyl group.

3. Process according to claim 1 or 2, **characterised in that** the base is an alkali- or earth alkali acetate, -carbonate, -hydrogencarbonate, -phosphate, or -hydrogenphosphate.

4. Process according to one of claims 1 to 3, **characterised in that** palladium in the form of bis(triphenylphosphine)palladium(II)chloride or palladium(II)acetate is used.

5. Process according to one of claims 1 to 4, **characterised in that** the bisdiphenylphosphine, is 1,1'-bis(diphenylphosphino)ferrocene or 1,4-bis(diphenylphosphino)butane.

6. Process according to one of claims 1 to 5, **characterised in that** the carbon monoxide pressure is 1 to 200 bar.

7. Process according to one of claims 1 to 6, **characterised in that** the reaction temperature is 100 to 250°C.

## Revendications

1. Procédé de préparation d'esters d'acides 2,6-pyridinedicarboxyliques de formule générale 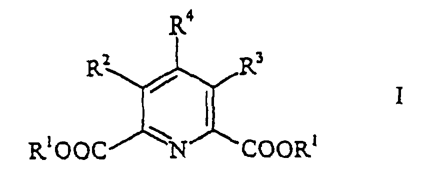 dans laquelle R¹ représente un groupe alkyle en C₁-C₆, un groupe cycloalkyle en C₃-C₆ ou un groupe aryle ou arylalkyle éventuellement substitué par alkyle en C₁-C₆ halogéné ou non halogéné, alcoxy en C₁-C₆, alkylthio en C₁-C₆, alcanesulfonyle en C₁-C₆ ou fluoro, R² et R³ représentent, indépendamment l'un de l'autre, un atome d'hydrogène ou de chlore, et R⁴ représente un atome d'hydrogène, un groupe alkyle en C₁-C₆, un groupe alcoxy en C₁-C₆ ou un atome de fluor, **caractérisé en ce que** l'on fait réagir une halogénopyridine de formule générale 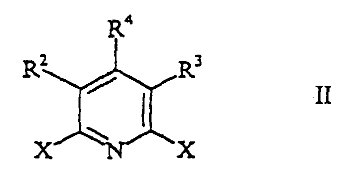 dans laquelle R², R³ et R⁴ ont la signification indiquée ci-dessus et X est un atome de chlore ou de brome, avec du monoxyde de carbone et un alcool de formule générale
R¹ - OH III
dans laquelle R¹ a la signification indiquée ci-dessus, en présence d'une base et d'un complexe de palladium avec une bis-diphénylphosphine de formule générale 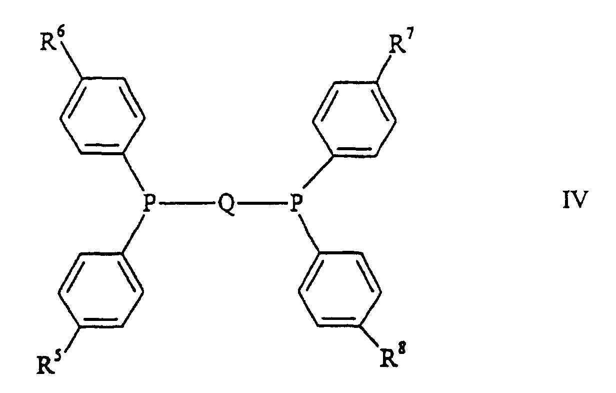 dans laquelle Q représente un groupe alcanediyle en C₃-C₆ ou un groupe 1,1'-ferrocènediyle dont les groupes cyclopentadiényle sont éventuellement substitués par des groupes alkyle en C₁-C₄ ou aryle, et les R⁵ à R⁸ représentent indépendamment les uns des autres un atome d'hydrogène ou un groupe alkyle en C₁-C₄, alcoxy en C₁-C₄, monofluorométhyle, difluorométhyle, trifluorométhyle, fluoro, aryle, aryloxy, cyano ou dialkylamino.

2. Procédé selon la revendication 1, **caractérisé en ce que** R¹ est un groupe méthyle, un groupe éthyle, un groupe butyle ou un groupe cyclohexyle.

3. Procédé selon la revendication 1 ou 2, **caractérisé en ce que** la base est un acétate, un carbonate, un hydrogénocarbonate, un phosphate ou un hydrogénophosphate de métal alcalin ou de métal alcalino-terreux.

4. Procédé selon l'une des revendications 1 à 3, **caractérisé en ce que** l'on utilise le palladium sous forme de chlorure de bis(triphénylphosphine)palladium (II) ou d'acétate de palladium (II).

5. Procédé selon l'une des revendications 1 à 4, **caractérisé en ce que** la bis-diphénylphosphine est le 1,1'-bis(diphénylphosphino)ferrocène ou le 1,4 bis(diphénylphosphino)butane.

6. Procédé selon l'une des revendications 1 à 5, **caractérisé en ce que** la pression de monoxyde de carbone est de 1 à 200 bars.

7. Procédé selon l'une des revendications 1 à 6, **caractérisé en ce que** la température de la réaction est de 100 à 250°C.
